# EUROPÄISCHE PATENTSCHRIFT

(11) **EP 3 521 267 B1**
(45) Veröffentlichungstag und Bekanntmachung des Hinweises auf die Patenterteilung: **30.12.2020**
(21) Anmeldenummer: 18020582.5
(22) Anmeldetag: 07.11.2018
(51) Int. Cl.: C07C 41/01, C07C 43/04, C07C 29/151, C07C 41/09, C07C 31/04, C07C 41/42, C07C 29/15

(54) **VERFAHREN ZUR GEWINNUNG VON DIMETHYLETHER UND KOHLENDIOXID**
METHOD FOR THE PRODUCTION OF DIMETHYL ETHER AND CARBON DIOXIDE
PROCÉDÉ D'OBTENTION DE DIMÉTHYLÉTHER ET DE DIOXYDE DE CARBONE

(30) Priorität: 22.11.2017 DE 102017010788
(43) Veröffentlichungstag der Anmeldung: 07.08.2019
(73) Patentinhaber: Linde GmbH, 82049 Pullach (DE)
(72) Erfinder: Peschel, Andreas, 82515 Wolfratshausen (DE)
(74) Vertreter: Fischer, Werner

(56) Entgegenhaltungen:
- WO-A1-2015/104238
- WO-A1-2015/104290
- WO-A1-2015/106952

## Beschreibung

Die Erfindung betrifft ein Verfahren zur Gewinnung von Dimethylether und Kohlendioxid gemäß den Patentansprüchen.

### Stand der Technik

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether wird herkömmlicherweise aus Synthesegas über das Zwischenprodukt Methanol gewonnen, das abgetrennt und weiter zu Dimethylether dehydratisiert wird. Die vorliegende Erfindung betrifft die hingegen die einstufige bzw. direkte Gewinnung von Dimethylether aus Synthesegas. Unter einer direkten Gewinnung wird hier eine Gewinnung ohne zwischengeschaltete Methanolabtrennung verstanden, wie sie in den herkömmlichen Verfahren erfolgt. Die bei der direkten Gewinnung von Dimethylether aus Synthesegas ablaufenden Reaktionen können ebenfalls Methanol als Zwischenprodukt liefern, das jedoch nicht abgetrennt wird sondern zumindest zum Teil sofort weiter zu Dimethylether reagiert. Entsprechende Verfahren sind seit längerem bekannt und werden auch unten näher erläutert.

Das Synthesegas wiederum kann mittels bekannter Verfahren, beispielsweise Dampfreformierung, aus Erd- oder Biogas erzeugt werden. Auch andere Verfahren zur Gewinnung von Synthesegas, beispielsweise durch Pyrolyse von Abfällen, Biomasse oder anderer organischer Materialien, sind bekannt.

Aus der WO 2015/106952 A1 ist ein Verfahren zur Herstellung von Dimethylether bekannt, wobei aus Synthesegas Kohlendioxid abgetrennt wird, um ein vorwiegend aus Kohlenmonoxid und Wasserstoff bestehendes Einsatzgas zu erhalten, das nachfolgend in einem einstufigen Prozess zu Dimethylether umgesetzt wird. Nötigenfalls wird Wasserstoff aus dem Synthesegas abgetrennt, um das Einsatzgas mit einem definierten molaren Verhältnis von Wasserstoff zu Kohlenmonoxid zu erhalten.

Die WO 2015/104290 A1 offenbart ein Verfahren zur trenntechnischen Bearbeitung eines Gasgemischs, das aus einem Produktstrom eines Reaktors zur Synthese von Dimethylether aus Synthesegas gebildet wird, und das zumindest Dimethylether, Kohlendioxid und zumindest eine weitere, leichter als Kohlendioxid siedende Komponente enthält.

Es besteht weiterhin der Bedarf nach verbesserten Verfahren zur Herstellung von Dimethylether, insbesondere nach solchen Verfahren, in denen sich alternative, in anderen großtechnischen Prozessen als Nebenprodukte anfallende Gase oder Gasgemische nutzen und insbesondere weitere Produkte herstellen lassen.

### Offenbarung der Erfindung

Vor diesem Hintergrund schlägt die vorliegende Erfindung ein Verfahren wie im unabhängigen Anspruch 1 definiert vor. Bevorzugte Ausführungsformen sind Gegenstand der Unteransprüche sowie der nachfolgenden Beschreibung.

Wie bereits erläutert, wird Dimethylether herkömmlicherweise durch die Dehydratisierung von Methanol erzeugt, wobei zur Herstellung des Methanols Synthesegas eingesetzt wird. Das für die Methanolherstellung eingesetzte Synthesegas zeichnet sich dabei durch eine Stöchiometriezahl (engl. Stoichiometric Number, SN) des als Frischeinsatz verwendeten Synthesegases, die geringfügig oberhalb von 2 liegt, aus. Die Stöchiometriezahl drückt die Zusammensetzung des Synthesegases hinsichtlich der Komponenten Wasserstoff, Kohlendioxid und Kohlenmonoxid aus, wobei jeweils die Stoffmengenanteile x betrachtet werden. Sie berechnet sich zu SN = (xH₂ - xCO₂) / (xCO + xCO₂).

Entsprechende Verfahren werden industriell eingesetzt. Für weitere Details hinsichtlich entsprechender Verfahren und Verfahrensvarianten sei auf einschlägige Fachliteratur, beispielsweise die Veröffentlichung "Dimethyl Ether Technology and Markets", CHEMYSTEMS PERP Program, Nexant Inc., November 2008, und das DME Handbook des Japan DME Forum, ISBN 978-4-9903839-0-9, 2007, verwiesen.

Alternativ kann Dimethylether, wie ebenfalls erwähnt, direkt aus Synthesegas gewonnen werden. Dazu werden die Katalysatoren zur Methanol- und Dimethylethersynthese in einem Reaktor kombiniert. Details hierzu sind beispielsweise ebenfalls im DME-Handbook des Japan DME Forum, bei Ogawa, T. et al., "Direct Dimethyl Ether Synthesis", J. Nat. Gas Chem. 2003, 12, 219-227, Lee, S.-H., "Scale Up Study of DME Direct Synthesis Technology", In: Proceedings of the 24th World Gas Conference, Buenos Aires, Argentina, 2009, und der WO 2015/104238 A1 angegeben.

Auch hier wird zunächst ein Synthesegas aus einem kohlenstoffhaltigen Einsatzstoff, beispielsweise Erdgas, durch bekannte Technologien hergestellt und anschließend direkt zu Dimethylether umgesetzt. Je nach der Stöchiometriezahl des jeweils frisch eingesetzten Synthesegases entstehen entweder Dimethylether und Wasser (SN ≥ 2) oder Dimethylether und Kohlendioxid (SN = 1).

In der direkten Dimethylethersynthese setzen sich Wasserstoff und Kohlenmonoxid entsprechend folgenden Reaktionen zu Methanol (MeOH) und Dimethylether um:

2 H₂ + CO ⇆ MeOH (1)

2 MeOH ⇆ DME + H₂O (2)

CO + H₂O ⇆ CO₂ + H₂ (3)

Es handelt sich hierbei um Gleichgewichtsreaktionen, die je nach Druck, Temperatur und Einsatzzusammensetzung eine andere Produktzusammensetzung ergeben. Bei einer Stöchiometriezahl von 1 beobachtet man die folgende Nettoreaktion:

3 H₂ + 3 CO ⇆ DME + CO₂ (4)

Bei einer Stöchiometriezahl von größer als 2 beobachtet man hingegen folgende Nettoreaktion:

2 H₂ + CO ⇆ DME + H₂O (5)

Bei zwischen den genannten Werten liegenden Stöchiometriezahlen bilden sich Wasser und Kohlendioxid parallel. Kohlendioxid wird in herkömmlichen Verfahren typischerweise mittels einer chemischen oder physikalischen Wäsche abgetrennt und kann zur Synthesegaserzeugung rezykliert werden.

Die vorliegende Erfindung beruht nun insbesondere auf der Erkenntnis, dass beispielsweise in Hüttenwerksprozessen anfallende Gase bzw. Gasgemische anstelle von Synthesegas ebenfalls besonders vorteilhaft zur Herstellung von Dimethylether durch Direktsynthese nutzen lassen, wenn sie die erforderlichen Komponenten Wasserstoff und Kohlenmonoxid enthalten. Wie erfindungsgemäß erkannt wurde, lässt sich in diesem Zusammenhang insbesondere reines bzw. hochreines Kohlendioxid als weiteres Verfahrensprodukt gewinnen. Insbesondere in integrierten Hüttenwerken werden häufig unterschiedliche, an Kohlenmonoxid und Wasserstoff reiche Gase bzw. Gasgemische gebildet, beispielsweise Koksofengas (häufig reich an Wasserstoff) und Hochofengas (häufig reich an Kohlenmonoxid). Diese Gase bzw. Gasgemische werden in Hüttenwerken häufig zur Wärmeintegration und zur Stromerzeugung durch Verbrennung in einer Gasturbine genutzt.

Werden, wie in der vorliegenden Erfindung in einer Ausgestaltung vorgeschlagen, entsprechende Gase bzw. Gasgemische stattdessen zur Herstellung von Syntheseprodukten, im vorliegenden Fall Dimethylether, genutzt, lassen sich bei der herkömmlicherweise stattfindenden Verbrennung freiwerdende Kohlendioxidemissionen reduzieren. Gleichzeitig kann der Verbrauch an fossilen kohlenstoffhaltigen Einsatzstoffen für die Synthesegasherstellung und die Herstellung der daraus gewonnenen Produkte entsprechend verringert werden.

Die genannten Gase oder Gasgemische oder wiederum aus diesen gebildeten Gemische weisen typischerweise eine Stöchiometriezahl auf, die deutlich unterhalb von 2 liegt. Wie bereits oben erläutert, wird daher bei der direkten Herstellung von Dimethylether Kohlendioxid in nicht unbeträchtlichem Umfang gebildet. Es fehlt allerdings hier an der Möglichkeit, dieses Kohlendioxid in eine (nicht vorhandene) Synthesegaserzeugung zurückzuführen.

Daher schlägt die vorliegende Erfindung vor, das bei der direkten Dimethylethersynthese aus einem entsprechenden alternativen Einsatz, der aus einem oder mehreren Hüttenwerksprozessen, aber auch aus anderen Prozessen stammen kann, gebildete Kohlendioxid abzutrennen und als weiteres Produkt bereitzustellen. Die vorliegende Erfindung zeigt dabei eine Möglichkeit auf, mittels derer es vergleichsweise einfach möglich ist, hochreines Kohlendioxid, das beispielsweise für die Verwendung in der Lebensmittelindustrie oder in chemischen Synthesen geeignet ist, zu erzeugen.

Durch die erfindungsgemäß vorgeschlagene gezielte Ausschleusung von Kohlendioxid können andere in der direkten Dimethylethersynthese umsetzbare Komponenten in diese zurückgeführt werden, wodurch sich die Stöchiometriezahl in dem der direkten Dimethylethersynthese zugeführten Reaktionseinsatz gegenüber dem jeweiligen Frischeinsatz erhöhen bzw. in der nachfolgend erläuterten Weise günstig beeinflussen lässt. Dies führt zu dem weiteren Vorteil einer deutlich besseren Stabilität des Katalysators in der direkten Dimethylethersynthese.

Insgesamt lassen sich durch den Einsatz der vorliegenden Erfindung zur Herstellung von Dimethylether Gase oder Gasgemische als Frischeinsätze nutzen, in denen das molare Verhältnis von Wasserstoff zu Kohlenmonoxid (das bei Abwesenheit von Kohlendioxid der Stöchiometriezahl entspricht) deutlich kleiner als 2 ist, und in denen geringe Mengen, beispielsweise weniger als 1.000 ppm, Kohlendioxid vorliegen. Beispielsweise kann das molare Verhältnis von Wasserstoff zu Kohlenmonoxid bzw. die Stöchiometriezahl im Rahmen der Erfindung bei ca. 1,15 liegen.

Die vorliegende Erfindung geht von einem Verfahren zur Herstellung von Dimethylether aus, bei dem ein Wasserstoff und Kohlenmonoxid enthaltender Reaktionseinsatz gebildet und der Reaktionseinsatz unter Erhalt eines Dimethylether, Wasser, Methanol, Kohlendioxid, Kohlenmonoxid und Wasserstoff enthaltenden Rohprodukts einer Dimethylether-Direktsynthese unterworfen wird. Das Rohprodukt kann neben den spezifisch erwähnten Komponenten auch weitere Komponenten, insbesondere Nebenprodukte der Dimethylether-Direktsynthese, enthalten, die in der Trennung jeweils ihrer Flüchtigkeit nach in die entsprechenden Fraktionen übergehen. Hierbei handelt es sich insbesondere um geringere Mengen an Methan, Ethan, organischen Säuren und höheren Alkoholen.

Unter einem "Reaktionseinsatz" wird dabei hier das direkt dem oder den in der Dimethylether-Direktsynthese eingesetzten Reaktoren zugeführte Gasgemisch verstanden, also das Gasgemisch, das in dem oder den Reaktoren umgesetzt wird. Der Reaktionseinsatz wird dabei aus einem Frischeinsatz und einem oder mehreren rückgeführten Gasen oder Gasgemischen bzw. Fluiden gebildet, wie auch nachfolgend noch im Detail erläutert. Ein "Frischeinsatz" oder "Makeup" stellt dabei ein Gas oder Gasgemisch dar, das nicht aus einem stromab des oder der Reaktoren, also der Dimethylether-Direktsynthese, vorliegenden Gasgemisch abgetrennte Anteile aufweist, also aus einem anderen chemischen oder verfahrenstechnischen Prozess stammt. Mit anderen Worten umfasst der Frischeinsatz keine in der Dimethylether-Direktsynthese gebildete Anteile.

Wie bereits erwähnt, kann im Rahmen der vorliegenden Erfindung insbesondere vorgesehen sein, einen entsprechenden Frischeinsatz aus einem oder mehreren Gasgemischen zu bilden, die in einem oder mehreren Hüttenwerksprozessen anfallen, beispielsweise aus Koksofen- und/oder Hochofengas. Es versteht sich, dass das oder die Gasgemische vor einem entsprechenden Einsatz im Rahmen der vorliegenden Erfindung einer geeigneten Aufbereitung, beispielsweise Trocknung, Entfernung von typischen Verunreinigungen des Hüttenprozesses, Entschwefelung, Wassergas-Shift-Reaktion, Sauergasentfernung und dergleichen unterworfen werden können, wie sie grundsätzlich aus dem Stand der Technik bekannt ist.

In dem im Rahmen der vorliegenden Erfindung eingesetzten Verfahren wird das Rohprodukt oder ein Teil hiervon als Trenneinsatz einer Trennung unterworfen, in der unter Abtrennung zumindest des überwiegenden Anteils des Dimethylethers, des Wassers und des Methanols sowie eines geringeren Anteils des Kohlenmonoxids und des Wasserstoffs aus dem Trenneinsatz ein Kohlendioxid, Kohlenmonoxid und Wasserstoff enthaltendes und an Dimethylether, Wasser und Methanol armes oder freies Zwischengemisch gebildet wird. Das Rohprodukt kann also im Rahmen der vorliegenden Erfindung vollständig oder nur zu einem definierten Teil (nämlich in Form des Trenneinsatzes) der Trennung unterworfen werden. In der Trennung werden dann zumindest der überwiegende Anteil des Dimethylethers, des Wassers und des Methanols in nachfolgend anhand eines Beispiels erläuterten Trennschritten, insbesondere durch eine Kombination rein destillativer und kondensativer Trennschritte, abgetrennt und insbesondere als getrennte oder gemeinsame Fraktionen gewonnen.

Das Zwischengemisch kann dabei bereits reich an Kohlendioxid sein und in entsprechender Weise gebildet werden. Der Gehalt an Kohlendioxid in dem Zwischengemisch kann bei mehr als 95 Molprozent, insbesondere mehr als 96, 97 oder 98 Molprozent, liegen. Der Gehalt an Dimethylether beträgt weniger als 1 Molprozent, insbesondere weniger als 0,1 Molprozent. Wasser und Methanol sind allenfalls in Spuren, d.h. zu weniger als 0,1 Molprozent, insbesondere zu weniger als 0,01 Molprozent, enthalten.

Fluide beliebiger Art werden hier generell als "reich" an einer oder mehreren enthaltenen Komponenten bezeichnet, wenn der Gehalt an der einen oder den mehreren Komponenten wenigstens 50%, 60%, 70%, 80%, 90%, 95%, 99% oder 99,5% auf molarer, Gewichts- oder Volumenbasis beträgt. Sie werden hingegen als "arm" an der einen oder den mehreren Komponenten bezeichnet, wenn der Gehalt an der einen oder den mehreren Komponenten höchstens 30%, 20%, 10%, 5%, 1% oder 0,5% auf molarer, Gewichts- oder Volumenbasis beträgt. Sie können im hier verwendeten Sprachgebrauch "angereichert" oder "abgereichert" an einer oder mehreren Komponenten sein, wobei sich diese Begriffe auf einen entsprechenden Gehalt in einem Ausgangsfluid beziehen, aus dem das jeweilige Fluid gebildet wurde. Das Fluid wird als angereichert bezeichnet, wenn es zumindest den 2-fachen, 5-fachen, 10-fachen, 100-fachen oder 1.000-fachen Gehalt der einen oder der mehreren Komponenten, bezogen auf das Ausgangsfluid, aufweist. Das Fluid wird hingegen als abgereichert bezeichnet, wenn es höchstens den 0,5-fachen, 0,1-fachen, 0,01-fachen oder 0,001-fachen Gehalt der einen oder der mehreren Komponenten, bezogen auf das Ausgangsfluid, aufweist. Ein "überwiegend" eine oder mehrere Komponenten enthaltendes Fluid ist reich an dieser oder diesen im Sinne der obigen Definition.

Ein Fluid (die Bezeichnung Fluid wird stellvertretend auch für entsprechende Ströme, Fraktionen usw. verwendet) ist im hier verwendeten Sprachgebrauch von einem anderen Fluid (das hier auch als Ausgangsfluid bezeichnet wird) abgeleitet oder aus einem solchen Fluid gebildet, wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Stoffstrom kann auch beispielsweise einfach dadurch gebildet werden, dass er aus einem Speicherbehälter abgezogen bzw. von einem anderen Stoffstrom abgetrennt wird.

Erfindungsgemäß ist vorgesehen, dass das Zwischengemisch in der Trennung flüssig bereitgestellt wird, dass unter Abtrennung zumindest des überwiegenden Anteils des Kohlendioxids aus dem Zwischengemisch oder einem Teil hiervon und unter Erhalt einer überwiegend oder ausschließlich Kohlendioxid enthaltenden Kohlendioxidfraktion ein Kohlenmonoxid und Wasserstoff enthaltendes und an Kohlendioxid armes oder freies Restgemisch gebildet wird, dass zumindest das Restgemisch oder ein Teil hiervon bei der Bildung des Reaktionseinsatzes verwendet wird, und dass der Reaktionseinsatz derart gebildet wird, dass ein molares Verhältnis von Wasserstoff zu Kohlenmonoxid in dem Reaktionseinsatz weniger als 2, insbesondere weniger als 1,8, oder 1,6 und mehr als 1,2 oder 1,4 beträgt. Der Reaktionseinsatz weist dabei insbesondere einen Kohlendioxidgehalt von 2 bis 10 Molprozent Kohlendioxid, insbesondere 6 bis 8 Molprozent Kohlendioxid, auf. Der Kohlendioxidgehalt in dem Trenneinsatz ergibt sich dabei durch einen Gehalt an Kohlendioxid in einem oder mehreren, nachfolgend noch im Detail erläuterten, rückgeführten Gasen oder Gasgemischen, das oder die bei der Bildung des Reaktionseinsatz verwendet werden. Letzterer Gehalt an Kohlendioxid wiederum wird insbesondere durch die Trennbedingungen in der mehrfach erwähnten Trennung, insbesondere die minimale Temperatur, die in der Trennung eingesetzt wird, beeinflusst. Auch letzteres wird nachfolgend noch erläutert.

Ein wesentlicher Aspekt der vorliegenden Erfindung ist der, dass das Zwischengemisch und die Kohlendioxidfraktion ohne Einsatz einer chemischen oder physikalischen Wäsche, wie sie herkömmlicherweise zur Abtrennung von Kohlendioxid zum Einsatz kommt, gebildet werden. Mit anderen Worten erfolgt die Trennung, in der unter Abtrennung zumindest des überwiegenden Anteils des Dimethylethers, des Wassers und des Methanols sowie eines geringeren Anteils des Kohlenmonoxids und des Wasserstoffs aus dem Trenneinsatz das Kohlendioxid, Kohlenmonoxid und Wasserstoff enthaltende und an Dimethylether, Wasser und Methanol arme oder freie Zwischengemisch gebildet wird, rein kondensativ und/oder destillativ. Es versteht sich jedoch, dass bei einer destillativen Trennung Kondensate aus einer Trennkolonne in diese zurückgeführt werden und zur Auswaschung von Komponenten aus einem in der Trennkolonne vorliegenden Gasgemisch verwendet werden können. Im Gegensatz zu einer physikalischen Wäsche werden hierbei jedoch keine Waschmittel eingesetzt, die nicht bereits in dem Trenneinsatz bzw. dem Rohprodukt enthalten waren. Auch die Bildung des Trenneinsatzes unter Verwendung des Rohprodukts oder eines entsprechenden Anteils des Rohprodukts erfolgt insbesondere ohne Einsatz einer entsprechenden chemischen oder physikalischen Wäsche. Ebendies gilt auch für die Bildung der Kohlendioxidfraktion. Auf diese Weise kann im Rahmen der vorliegenden Erfindung die Bildung der Kohlendioxidfraktion einfach und im Vergleich zu herkömmlicherweise verwendeten chemischen oder physikalischen Wäschen mit ausgesprochen geringem Zusatzaufwand durchgeführt werden.

Durch die Kombination der erfindungsgemäßen Maßnahmen werden die bereits zuvor erläuterten Vorteile erzielt. Die flüssige Bereitstellung des Zwischengemischs, insbesondere als Kondensat aus einem Kopfkondensator einer stromauf angeordneten Trennkolonne, kann das Kondensat in einer besonders einfach und kostengünstig bereitstellbaren, weil vergleichsweise einfach aufgebauten, weiteren Trennkolonne aufgetrennt werden, die mit einer geringen Bodenzahl und ohne Kopfkondensator ausgebildet sein kann. In dieser werden im Wesentlichen noch die in der Zwischenfraktion enthaltenen leichteren Komponenten wie Kohlenmonoxid und Wasserstoff aus dieser Zwischenfraktion abgetrennt. Alternativ kann das flüssig bereitgestellte Zwischengemisch auch lediglich einer Entspannung unterworfen werden, bei der leichter als das Kohlendioxid flüchtige Komponenten aus dem Zwischengemisch verdampft werden bzw. ausgasen.

Durch die Entfernung von Kohlendioxid aus einer im Übrigen in die Dimethylether-Direktsynthese rückgeführten Fraktion wird in einem entsprechend mit den anderen Komponenten gebildeten Kreislauf um den oder die Synthesereaktoren das molare Verhältnis von Wasserstoff zu Kohlenmonoxid im Reaktionseinsatz erhöht, beispielsweise von einem Wert von ca. 1,15 auf einen Wert von ca. 1,5. Die Stöchiometriezahl kann hingegen, je nach dem Gehalt an Kohlendioxid in einem oder mehreren rückgeführten Gasen oder Gasgemischen, ggf. sogar verringert. Wie erwähnt, kann auf diese Weise ein stabilerer Betrieb des verwendeten Katalysators erzielt werden, weil dessen Langzeitstabilität gegen Alterung erhöht werden kann. Dies gilt insbesondere für die erwähnten Kohlendioxidgehalte von 2 bis 10 Molprozent. Entsprechende Zusammensetzungen führen ferner zu einer verbesserten Selektivität und Reaktionsgeschwindigkeit.

Das im Rahmen der vorliegenden Erfindung hergestellte Kohlendioxid kann insbesondere, wie erwähnt, für Lebensmittelzwecke eingesetzt werden. Es ist auch möglich, das Kohlendioxid teilweise oder vor oder alternativ zu einer entsprechenden Verwendung als Produkt als Kältemittel in einen Kältemittelkreislauf einzuspeisen und zur Bereitstellung von Prozesskälte zu verwenden. In einer derartigen Verfahrensvariante kann ein externes Prozessmedium ggf. in entsprechendem Umfang reduziert oder vollständig eingespart werden. Anwendungsgebiete des erfindungsgemäß hergestellten Kohlendioxids umfassen den Einsatz in chemischen Prozessen, Gewächshäusern und der Erdölgewinnung (sogenannte Enhanced Oil Recovery). Entsprechendes Kohlendioxid kann auch gespeichert und beispielsweise in Gesteinsschichten verpresst werden (sogenannte Carbon Capture and Storage).

Wie bereits erwähnt, wird im Rahmen der vorliegenden Erfindung insbesondere eine definierte Menge an Kohlendioxid in das Verfahren zurückgeführt, wodurch sich die erwähnten Gehalte in dem Reaktionseinsatz ergeben. Dies kann insbesondere durch eine Einstellung von zur Abtrennung von Kondensaten verwendeten Temperaturen in einem verwendeten Trennprozess erfolgen, insbesondere der dort verwendeten Minimaltemperatur. In dem in der Figur dargestellten Beispiel richtet sich der Gehalt an Kohlendioxid in dem rückgeführten Stoffstrom Q insbesondere nach den Temperaturen, die in dem Wärmetauscher stromauf des Abscheiders S3 eingesetzt werden.

Durch den Einsatz des erfindungsgemäßen Verfahrens wird es möglich, die in einer entsprechenden Anlage verwendeten Apparate (Reaktor oder Reaktoren, Verdichter, Trennkolonnen) wesentlich kleiner als in einer konventionellen Anordnung zur Direktsynthese von Dimethylether, in der ein Frischeinsatz mit einer Stöchiometriezahl von 2 bzw. einem entsprechend höheren molaren Verhältnis von Wasserstoff zu Kohlenmonoxid verwendet wird, auszubilden. Dies ist deshalb der Fall, weil der Reaktionsumsatz durch das geringe molare Verhältnis von Wasserstoff zu Kohlenmonoxid und den geringen Anteil an Kohlendioxid im Reaktionseinsatz gegenüber einem Synthesegas mit einer Stöchiömetriezahl von 2 wesentlich erhöht wird. Dadurch reduziert sich die Menge an zurückgeführtem Synthesegas signifikant. Im Gegensatz zur thermischen Verwertung von Hüttengasen, wie sie zuvor erläutert werden, können im Rahmen der vorliegenden Erfindung die Emissionen des Gesamtverfahrens deutlich reduziert werden, da das entstehende Kohlendioxid nicht in die Atmosphäre emittiert wird. Entsprechends gilt auch für andere in der vorliegenden Erfindung als Frischeinsätze einsetzbare Gase oder Gasgemische.

Besonders vorteilhaft ist das erfindungsgemäße Verfahren, wie mehrfach erwähnt, wenn bei der Bildung des Reaktionseinsatzes ferner ein Wasserstoff und Kohlenmonoxid enthaltender und an Kohlendioxid armer oder freier Frischeinsatz verwendet wird, wobei ein molares Verhältnis von Wasserstoff zu Kohlenmonoxid in dem Frischeinsatz 1,0 bis 1,8, insbesondere 1,05 bis 1,6, vorzugsweise 1,1 bis 1,3, beträgt. Da der Frischeinsatz an Kohlendioxid arm oder frei ist, entspricht das molare Verhältnis von Wasserstoff zu Kohlenmonoxid (im Wesentlichen oder vollständig) der Stöchiometriezahl.

Derartige Frischeinsätze können, wie erwähnt, insbesondere in Form von Gasen oder Gasgemischen vorliegen, die mittels eines oder mehrerer Hüttenwerksprozesse bereitgestellt wird oder werden. Die zumindest teilweise Bereitstellung des Frischeinsatzes mittels eines oder mehrerer Hüttenwerksprozesse, insbesondere mittels eines Hochofen- und eines Koksofenprozesses, stellt daher eine besonders bevorzugte Ausführungsform der vorliegenden Erfindung dar.

Gemäß einer weiteren Ausführungsform der vorliegenden Erfindung kann der Frischeinsatz jedoch auch (alternativ oder zu einem Anteil) unter Einsatz eines Kohlevergasungsprozesses bereitgestellt werden. In derartigen Kohlevergasungsprozessen wird ein Synthesegas hergestellt, das je nach der eingesetzten Prozessvariante (insbesondere bei der Trockenvergasung) ein molares Verhältnis von Wasserstoff zu Kohlenmonoxid von ca. 1:3 aufweist. Zum Einsatz als oder als Teil des erfindungsgemäß verwendeten Reaktionseinsatzes wird dieses Synthesegas dabei einer Wassergasshift unterworfen und anschließend, beispielsweise in einer Wäsche, insbesondere einer Rectisolwäsche, von Schwefelkomponenten und Kohlendioxid befreit. Durch das geringe Verhältnis von Wasserstoff zu Kohlenmonoxid, das für den Einsatz im Rahmen der vorliegenden Erfindung erreicht werden muss (im Gegensatz zu dem Wert von 2 für herkömmliche Verfahren) können die für die Wassergasshift und die vorgeschaltete Wäsche eingesetzten Apparate daher deutlich kleiner ausgebildet werden, wodurch sich die Erstellungskosten und Emissionen an Kohlendioxid verringern.

Im Verfahren gemäß der vorliegenden Erfindung kann in der Trennung insbesondere auch eine überwiegend oder ausschließlich Methanol enthaltende Methanolfraktion gebildet und bei der Bildung des Reaktionseinsatzes verwendet werden. Auf diese Weise lässt sich auch das als Nebenprodukt in der Dimethylether-Direktsynthese gebildete Methanol vorteilhaft zu Dimethylether umsetzen. Wie erwähnt, wird auch in einer Dimethylether-Direktsynthese Dimethylether über das Zwischenprodukt Methanol synthetisiert, indem ein bifunktionaler Katalysator eingesetzt wird. Daher kann Methanol entsprechend umgesetzt werden.

In dem erfindungsgemäß durchgeführten Verfahren kann die Kohlendioxidfraktion derart gebildet werden, dass sie wenigstens 99, 99,9, 99,99 oder 99,995 Molprozent Kohlendioxid aufweist. Es kann also im Rahmen der vorliegenden Erfindung insbesondere auch sogenanntes "Kohlendioxid 4.5", das sich gemäß einschlägiger Normen wie DIN EN ISO 14175: C1 zur Verwendung in der Lebensmittelproduktion eignet, erzeugt werden.

Im Rahmen der vorliegenden Erfindung erfolgt die Abtrennung zumindest des überwiegenden Anteils des Kohlendioxids aus dem Zwischengemisch oder dessen Anteil dabei insbesondere unter Verwendung einer Kohlendioxidabtrennkolonne. Bei dieser Kohlendioxidabtrennkolonne kann es sich insbesondere um eine Trennkolonne handeln, die mit einem Sumpfverdampfer und ohne einen Kopfkondensator betrieben wird und sich daher einfach und kostengünstig erstellen lässt. Dabei kann im Rahmen der vorliegenden Erfindung auch eine Trennkolonne als die Kohlendioxidabtrennkolonne verwendet werden, die eine vergleichsweise geringe Anzahl an theoretischen Trennböden, insbesondere 5 bis 20 theoretische Trennböden, aufweist. Anstelle einer Kohlendioxidabtrennkolonne kann jedoch auch lediglich eine Einrichtung zur Entspannung des Zwischengemischs verwendet werden. Eine entsprechende Entspannung kann ausreichen, um die leichteren Gase in dem Zwischengemisch von dem Kohlendioxid abzutrennen.

In dem erfindungsgemäßen Verfahren wird der Trenneinsatz in der Trennung vorteilhafterweise in einer ersten Trennkolonne bearbeitet, in der eine an, Kohlendioxid, Kohlenmonoxid und Wasserstoff abgereicherte und Wasser und Methanol enthaltende erste Sumpfflüssigkeit und ein an, Kohlendioxid, Kohlenmonoxid und Wasserstoff angereichertes und an Wasser und Methanol armes oder freies erstes Kopfgas gebildet werden. Sind in dem Trenneinsatz weitere leichtere oder schwerere Komponenten enthalten, gehen diese entsprechend ihrer Flüchtigkeit in die erste Sumpfflüssigkeit oder das erste Kopfgas über. Dimethylether verteilt sich in der ersten Trennkolonne je nach den spezifisch verwendeten Trenneigenschaften zwischen dem Kopfgas und der Sumpfflüssigkeit, geht jedoch zu gewissen Anteilen sowohl in das Kopfgas als auch in die Sumpfflüssigkeit über.

Die erste Trennkolonne weist insbesondere einen Kopfkondensator auf, in dem beispielsweise Kühlwasser als Kältemittel verwendet werden kann, und mittels dessen ein Teil des ersten Kopfgases kondensiert und auf die erste Trennkolonne zurückgeführt werden kann. Die erste Trennkolonne wird insbesondere auf einem Druckniveau von 30 bis 100 bar (abs.) und auf einem Temperaturniveau von 10 bis 70 °C (am Kopf) betrieben.

In dem erfindungsgemäß vorgeschlagenen Verfahren wird die erste Sumpfflüssigkeit oder ein Teil hiervon vorteilhafterweise in der Trennung in einer zweiten Trennkolonne bearbeitet, in der eine Wasser und Methanol enthaltende und an Dimethylether, Kohlendioxid, Kohlenmonoxid und Wasserstoff arme oder freie zweite Sumpfflüssigkeit und ein Dimethylether, Kohlendioxid, Kohlenmonoxid und Wasserstoff enthaltendes und an Wasser und Methanol armes oder freies zweites Kopfgas gebildet werden. Analog zu den obigen Erläuterungen gilt, dass wenn in der ersten Sumpfflüssigkeit bzw. in deren in die zweite Trennkolonne überführtem Teil weitere leichtere oder schwerere Komponenten enthalten sind, diese entsprechend ihrer Flüchtigkeit in die zweite Sumpfflüssigkeit oder das zweite Kopfgas übergehen.

Die zweite Trennkolonne weist insbesondere einen Kopfkondensator auf, in dem Kühlwasser als Kältemittel verwendet werden kann, und mittels dessen ein Teil des zweiten Kopfgases kondensiert und auf die zweite Trennkolonne zurückgeführt werden kann. Die zweite Trennkolonne weist ferner insbesondere einen Sumpfverdampfer auf, in dem Dampf als Heizmedium verwendet werden kann, und mittels dessen ein Teil der zweiten Sumpfflüssigkeit verdampft und in die dritte Trennkolonne zurückgeführt werden kann. Die zweite Trennkolonne wird insbesondere auf einem Druckniveau von 15 bis 40 bar (abs.) und auf einem Temperaturniveau von 10 bis 100 °C (am Kopf) betrieben.

In dem erfindungsgemäß vorgeschlagenen Verfahren wird die zweite Sumpfflüssigkeit bzw. deren nicht verdampfter und in die zweite Trennkolonne rückgeführter Teil vorteilhafterweise in der Trennung in einer dritten Trennkolonne bearbeitet, in der eine überwiegend oder ausschließlich Wasser enthaltende dritte Sumpfflüssigkeit und ein überwiegend oder ausschließlich Methanol enthaltendes drittes Kopfgas gebildet werden. Das dritte Kopfgas kann insbesondere kondensiert und zu einem Teil auf die dritte Trennkolonne zurückgeführt werden. Ein weiterer Teil des kondensierten dritten Kopfgases kann insbesondere mittels einer Pumpe auf einen geeigneten Druck verdichtet, verdampft und in die Dimethylether-Direktsynthese zurückgeführt werden. Analog zu den obigen Erläuterungen gilt auch hier, dass wenn in der zweiten Sumpfflüssigkeit bzw. in deren in die dritte Trennkolonne überführtem Teil weitere leichtere oder schwerere Komponenten enthalten sind, diese entsprechend ihrer Flüchtigkeit in die dritte Sumpfflüssigkeit oder das dritte Kopfgas übergehen.

Die dritte Trennkolonne weist insbesondere einen Kopfkondensator auf, in dem Kühlwasser als Kältemittel verwendet werden kann, und mittels dessen ein Teil des dritten Kopfgases kondensiert und auf die dritte Trennkolonne zurückgeführt werden kann. Die dritte Trennkolonne weist ferner insbesondere einen Sumpfverdampfer auf, in dem Dampf als Heizmedium verwendet werden kann, und mittels dessen ein Teil der dritten Sumpfflüssigkeit verdampft und in die dritte Trennkolonne zurückgeführt werden kann. Die dritte Trennkolonne wird dabei insbesondere auf einem Druckniveau von 1 bis 10 bar (abs.) und auf einem Temperaturniveau von 30 bis 140 °C (am Kopf) betrieben.

Im Rahmen der vorliegenden Erfindung ist insbesondere vorgesehen, dass das erste Kopfgas oder ein Teil hiervon, insbesondere ein nicht kondensierter Anteil hiervon, unter Bildung von Kondensaten einer stufenweisen Abkühlung unterworfen wird. Die Abkühlung kann dabei insbesondere einen ersten Abkühlschritt auf ein Temperaturniveau von 20 bis -10 °C, in dem Kühlwasser, Kohlendioxid oder ein kohlenwasserstoffreiches Kältemittel wie Propan oder Propen als Kältemittel eingesetzt wird, einen zweiten Abkühlschritt auf ein Temperaturniveau von -10 bis -30 °C, in dem Kohlendioxid oder ein kohlenwasserstoffreiches Kältemittel wie Propan oder Propen als Kältemittel eingesetzt wird, und einen dritten Abkühlschritt auf ein Temperaturniveau von -20 bis -50 °C, in dem Kohlendioxid oder ein kohlenwasserstoffreiches Kältemittel wie Propan oder Propen als Kältemittel eingesetzt wird, umfassen. Nach jedem Abkühlschritt kann das entsprechend abgekühlte Fluid insbesondere in einen Abscheider eingespeist werden, dem eine flüssige und eine gasförmige Fraktion entnommen werden können. Die gasförmigen Fraktionen werden dabei jeweils dem nächsten Abkühlschritt zugeführt. Statt den erwähnten drei Abkühlschritten kann auch eine höhere oder geringere Anzahl an Abkühlschritten erfolgen, wobei jedoch insbesondere in dem letzten Abkühlschritt eine Temperatur ein Temperaturniveau von -55 °C nicht unterschritten wird, um kein festes Kohlendioxid zu erhalten.

Hingegen wird ein bei der stufenweisen Abkühlung des Kopfgases oder seines Anteils gasförmig verbleibender Rest, d.h. die auch nach dem letzten Abkühlschritt gasförmig verbleibende Fraktion, oder ein Teil hiervon, vorteilhafterweise bei der Bildung des Reaktionseinsatzes verwendet, also als Teil des Reaktionseinsatzes zur Dimethylether-Direktsynthese zurückgeführt. Wie erwähnt, richtet sich ein Gehalt an Kohlendioxid in der nach dem letzten Abkühlschritt gasförmig verbleibenden Fraktion nach den Temperaturen in der Trennung.

Im Rahmen der vorliegenden Erfindung werden die bei der stufenweisen Abkühlung gebildeten Kondensate oder ein Anteil hiervon und das zweite Kopfgas oder ein Anteil hiervon, insbesondere ein nicht kondensierter und auf die zweite Trennkolonne rückgeführter Anteil des zweiten Kopfgases, in einer vierten Trennkolonne bearbeitet, in der ein Kohlendioxid, Kohlenmonoxid und Wasserstoff enthaltendes und an Dimethylether armes oder freies viertes Kopfgas und eine überwiegend oder ausschließlich Dimethylether enthaltende vierte Sumpfflüssigkeit gebildet werden. Wiederum gilt auch hier, dass dann, wenn in den in die vierte Trennkolonne eingespeisten Fluiden leichtere oder schwerere Komponenten enthalten sind, diese in die vierte Sumpfflüssigkeit bzw. das vierte Kopfgas übergehen. Im Rahmen der vorliegenden Erfindung kann die vierte Trennkolonne auch derart betrieben werden, dass ein gewisser Anteil des Kohlendioxids in das vierte Kopfgas übergeht, so dass auch dieses in die Dimethylether-Direktsynthese zurückgeführt werden kann.

Im Rahmen der vorliegenden Erfindung wird insbesondere ein Teil des vierten Kopfgases kondensiert und zu einem Teil auf die vierte Trennkolonne zurückgeführt und zu einem Teil als das Zwischengemisch verwendet, wobei ein nicht kondensierter Teil des vierten Kopfgases oder ein Teil hiervon bei der Bildung des Reaktionseinsatzes verwendet wird. Auf diese Weise kann der flüssige Trenneinsatz für die Kohlendioxidabtrennung bereitgestellt werden, der sich besonders einfach und effizient in der Kohlendioxidabtrennkolonne bearbeiten lässt.

Die Erfindung wird unter Bezugnahme auf die beigefügte Zeichnung näher erläutert, die eine Ausführungsform der Erfindung veranschaulicht.

### Kurze Beschreibung der Zeichnungen

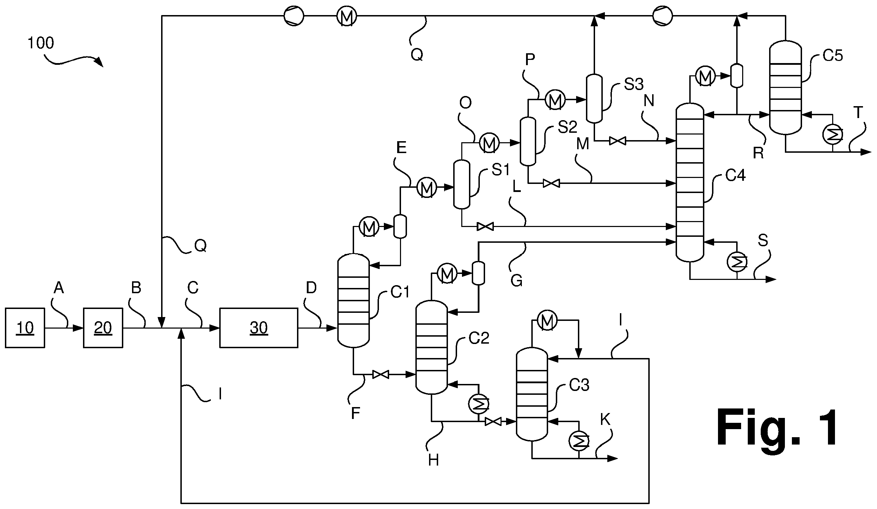

- Figur 1: veranschaulicht ein Verfahren gemäß einer Ausführungsform der Erfindung.

### Ausführliche Beschreibung der Zeichnungen

In Figur 1 ist ein Verfahren gemäß einer Ausführungsform der Erfindung in Form eines vereinfachten Prozessflussdiagramms schematisch veranschaulicht und insgesamt mit 100 bezeichnet.

In dem Verfahren 100 wird mittels eines oder mehrerer Hüttenwerksprozesse, der oder die hier insgesamt vereinfacht mit 10 bezeichnet ist oder sind, ein Stoffstrom A bereitgestellt. Wie hier vereinfacht mit 20 veranschaulicht, kann der Stoffstrom A auch in geeigneter Weise aufgereinigt werden, wodurch ein aufgereinigter Stoffstrom B erhalten wird. Der Stoffstrom B weist dabei in der hier veranschaulichten Ausführungsform ein molares Verhältnis von Wasserstoff zu Kohlenmonoxid von deutlich weniger als 2, beispielsweise von 1 bis 1,5 oder 1,1 bis 1,2, insbesondere von ca. 1,15, auf. Weitere mögliche Werte wurden bereits erläutert.

Der aufgereinigte Stoffstrom B wird im dargestellten Beispiel mit zwei rückgeführten Stoffströmen I und Q vereinigt, deren Zusammensetzung unten erläutert wird. Ein auf diese Weise gebildeter Einsatzstrom C wird einer Dimethylether-Direktsynthese 30 unter Verwendung eines oder mehrerer, mit geeigneten Katalysatoren ausgestatteter Reaktoren zugeführt.

In der Dimethylether-Direktsynthese 10 wird ein Rohproduktstrom D gewonnen, der, wie hier nicht gesondert veranschaulicht, abgekühlt wird. Die Abkühlung kann beispielsweise zumindest teilweise durch eine Wärmeintegration mit einem oder mehreren Stoffströmen, beispielsweise dem aufgereinigten Stoffstrom B oder dem Einsatzstrom C, durchgeführt werden. Der entsprechend abgekühlte Rohproduktstrom D wird in eine erste Trennkolonne C1 eingespeist.

Kopfgas der ersten Trennkolonne C1 wird abgekühlt und teilkondensiert. Der kondensierte Anteil des Kopfgases der ersten Trennkolonne C1 wird als Rücklauf auf die erste Trennkolonne C1 zurückgeführt. Der nicht kondensierte Anteil des Kopfgases der ersten Trennkolonne C1 wird in Form eines Stoffstroms E einer stufenweisen weiteren Abkühlung unterworfen, wie unten weiter erläutert. Sumpfflüssigkeit der ersten Trennkolonne C1 wird über ein nicht gesondert bezeichnetes Ventil in Form eines Stoffstroms F einer zweiten Trennkolonne C2 zugeführt. Die in Form des Stoffstroms F der zweiten Trennkolonne zugeführte Flüssigkeit enthält überwiegend oder ausschließlich Methanol und Wasser sowie Dimethylether und noch weitere gelöste Gase.

In der zweiten Trennkolonne C2 wird die in die zweite Trennkolonne C2 in Form des Stoffstroms F eingespeiste Flüssigkeit von Dimethylether und weiteren gelösten Gasen befreit, die in ein Kopfgas der zweiten Trennkolonne übergehen. Eine Sumpfflüssigkeit der zweiten Trennkolonne C2 enthält daher überwiegend oder ausschließlich Methanol und Wasser sowie ggf. schwerer siedende Komponenten. Sie wird in einem Sumpfverdampfer teilweise verdampft und gasförmig in die zweite Trennkolonne C2 zurückgeführt. Das Kopfgas der zweiten Trennkolonne C2 wird abgekühlt und teilkondensiert. Der kondensierte Anteil des Kopfgases der zweiten Trennkolonne C2 wird als Rücklauf auf die zweite Trennkolonne C2 zurückgeführt. Der nicht kondensierte Anteil des Kopfgases der zweiten Trennkolonne C2 wird in Form eines Stoffstroms G einer weiteren Trennung unterworfen, wie unten weiter erläutert. Die Sumpfflüssigkeit der zweiten Trennkolonne C2 bzw. deren nicht verdampfter und in die zweite Trennkolonne C2 zurückgeführter Anteil wird über ein nicht gesondert bezeichnetes Ventil in Form eines Stoffstroms H einer dritten Trennkolonne C3 zugeführt.

In der dritten Trennkolonne C3 wird die in die dritte Trennkolonne C3 in Form des Stoffstroms H eingespeiste Flüssigkeit in ein überwiegend oder ausschließlich Methanol enthaltendes Kopfgas und eine überwiegend oder ausschließlich Wasser enthaltende Sumpfflüssigkeit getrennt. Auch weitere schwerere Komponenten können enthalten sein. Das Kopfgas der dritten Trennkolonne C3 wird abgekühlt, kondensiert, und zu einem Teil als Rücklauf auf die dritte Trennkolonne C3 aufgegeben und zu einem weiteren Teil in Form des bereits erwähnten Stoffstroms I in das Verfahren 100 zurückgeführt. Wie erwähnt, aber nicht explizit veranschaulicht, kann dabei der Stoffstrom I flüssig auf Druck gebracht und verdampft werden. Die Sumpfflüssigkeit der dritten Trennkolonne C3 wird in zum Teil in einem Sumpfverdampfer verdampft und gasförmig in die dritte Trennkolonne C3 zurückgeführt und zum Teil in Form eines Stoffstroms K aus dem Verfahren 100 ausgeführt.

Das in Form des bereits erwähnten Stoffstroms E abgezogene Kopfgas der ersten Trennkolonne C1 enthält überwiegend oder ausschließlich Dimethylether, Kohlendioxid und leichter siedende gelöste Gase, insbesondere Wasserstoff und Kohlenmonoxid, ist jedoch arm an oder frei von Wasser und Methanol. Der Stoffstrom E wird einer stufenweisen Abkühlung zugeführt, wobei nach jeder Abkühlstufe jeweils in Abscheidern S1, S2 und S3 Kondensate abgeschieden und in Form von Stoffströmen L, M und N aus den Abscheidern S1, S2 und S3 abgezogen werden. Wie erwähnt, können statt den gezeigten drei Abkühl- und Kondensationsschritten auch mehr oder weniger Abkühl- und Kondensationsschritte zum Einsatz kommen. Die in den Abscheidern S1 und S2 gasförmig verbleibenden Anteile werden weiter abgekühlt und in Form von Stoffströmen O bzw. P dem jeweils nächsten Abscheider S2 bzw. S3 zugeführt. Ein auch in dem Abscheider S3 gasförmig verbleibender Anteil wird als Teil des bereits erwähnten Stoffstroms in das Verfahren 100 zurückgeführt. Der Stoffstrom Q kann zur Kälterückgewinnung in einem nicht gesondert bezeichneten Wärmetauscher genutzt werden und wird verdichtet, um ihn auf einem geeigneten Druck zu der Dimethylether-Direktsynthese 30 zurückzuführen.

Die in Form der erläuterten Stoffströme G, L, M und N bereitgestellten, an Dimethylether reichen Fluide, die zudem noch Kohlendioxid und leichter als Kohlendioxid und leichter siedende gelöste Gase, insbesondere Wasserstoff und Kohlenmonoxid, enthalten, jedoch arm an oder frei von Wasser und Methanol sind, werden zur Abtrennung von DME einer vierten Trennkolonne C4 zugeführt. In der vierten Trennkolonne C4 werden eine überwiegend oder ausschließlich Dimethylether enthaltende Sumpfflüssigkeit und ein überwiegend oder ausschließlich Kohlendioxid und leichter siedende Gase, insbesondere Wasserstoff und Kohlenmonoxid, enthaltendes Kopfgas gebildet. Das Kopfgas der vierten Trennkolonne C4 wird abgekühlt und teil kondensiert. Ein kondensierter Anteil des Kopfgases der vierten Trennkolonne C4 wird zu einem Teil als Rücklauf auf die vierte Trennkolonne C4 aufgegeben und zu einem Teil in Form eines Stoffstroms R in eine fünfte Trennkolonne C5 eingespeist. Ein nicht kondensierter Anteil des Kopfgases der vierten Trennkolonne C4 wird als Teil des bereits erwähnten Stoffstroms Q in das Verfahren 100 zurückgeführt. Die Sumpfflüssigkeit der vierten Trennkolonne C4 wird in zum Teil in einem Sumpfverdampfer verdampft und gasförmig in die vierte Trennkolonne C4 zurückgeführt und zum Teil in Form eines Stoffstroms S als Dimethylether-Produkt aus dem Verfahren 100 ausgeführt. Das Dimethylether-Produkt kann insbesondere Kraftstoffqualität aufweisen.

Das in Form des Stoffstroms R in die fünfte Trennkolonne C5 eingespeiste kondensierte Kopfgas der vierten Trennkolonne C4 wird in der fünften Trennkolonne C5 in eine überwiegend oder ausschließlich Kohlendioxid enthaltende Sumpfflüssigkeit und ein überwiegend oder ausschließlich die leichter siedenden Gase, insbesondere Wasserstoff und Kohlenmonoxid, enthaltendes Kopfgas aufgetrennt. Das Kopfgas kann als Teil des Stoffstroms Q in das Verfahren 100 zurückgeführt werden. Es kann dabei insbesondere mit dem nicht kondensierten Anteil des Kopfgases der vierten Trennkolonne C4 vereinigt und verdichtet werden, bevor es mit dem in dem Abscheider S3 gasförmig verbleibender Anteil vereinigt wird. Die Sumpfflüssigkeit der fünften Trennkolonne C5 wird zum Teil in einem Sumpfverdampfer verdampft und gasförmig in die fünfte Trennkolonne C5 zurückgeführt und zum Teil in Form eines Stoffstroms T als Kohlendioxidprodukt aus dem Verfahren 100 ausgeführt. Die fünfte Trennkolonne C5 kann vergleichsweise einfach ausgebildet sein und nur eine geringe Anzahl an Trennböden aufweisen. Auf einen Kopfkondensator kann verzichtet werden. Dennoch kann unter Verwendung der fünften Trennkolonne C5 das in Form des Stoffstroms T ausgeführte Kohlendioxidprodukt in hochreiner Form gewonnen und beispielsweise in Lebensmittelqualität als sogenanntes Kohlendioxid 4.5 bereitgestellt werden. Wie erwähnt kann alternativ zur Verwendung einer fünften Trennkolonne C5, die hier auch als Kohlendioxidabtrennkolonne bezeichnet wird, kann eine entsprechende Aufreinigung auch lediglich durch eine Entspannung mit anschließender Gas-Flüssig-Trennung erfolgen.

Durch die Entfernung des Kohlendioxids in der fünften Trennkolonne kann also der Vorteil einer vollständigen oder nahezu vollständigen Kohlendioxidentfernung unter gleichzeitiger Bereitstellung eines attraktiven Kohlendioxidprodukts erzielt werden. Weil lediglich die leichter siedenden Gase, insbesondere Wasserstoff und Kohlenmonoxid, sowie Methanol in das Verfahren 100, d.h. in die DME-Synthese 30, zurückgeführt werden, können die erwähnten Prozessbedingungen eingestellt werden. Insbesondere kann unter Verwendung des aufgereinigten Stoffstroms B mit den oben genannten Spezifikationen und die erläuterte Rückführung ein molares Verhältnis von Wasserstoff zu Kohlenmonoxid im erwähnten Bereich, insbesondere von ca. 1,5, eingestellt bzw. auf einen derartigen Wert erhöht werden.

## Patentansprüche

1. Verfahren (100) zur Herstellung von Dimethylether, bei dem ein Wasserstoff und Kohlenmonoxid enthaltender Reaktionseinsatz gebildet und der Reaktionseinsatz unter Erhalt eines Dimethylether, Wasser, Methanol, Kohlendioxid, Kohlenmonoxid und Wasserstoff enthaltenden Rohprodukts einer Dimethylether-Direktsynthese unterworfen wird, wobei das Rohprodukt oder ein Teil hiervon als Trenneinsatz einer Trennung unterworfen wird, in der unter Abtrennung zumindest des überwiegenden Anteils des Dimethylethers, des Wassers und des Methanols sowie eines geringeren Anteils des Kohlenmonoxids und des Wasserstoffs aus dem Trenneinsatz ein Kohlendioxid, Kohlenmonoxid und Wasserstoff enthaltendes und an Dimethylether, Wasser und Methanol armes oder freies Zwischengemisch gebildet wird, wobei die Trennung, in der das Zwischengemisch gebildet wird, rein kondensativ und/oder destillativ vorgenommen wird, **dadurch gekennzeichnet, dass** das Zwischengemisch in der Trennung flüssig bereitgestellt wird, dass unter Abtrennung zumindest des überwiegenden Anteils des Kohlendioxids aus dem Zwischengemisch oder einem Teil hiervon und unter Erhalt einer überwiegend oder ausschließlich Kohlendioxid enthaltenden Kohlendioxidfraktion ein Kohlenmonoxid und Wasserstoff enthaltendes und an Kohlendioxid armes oder freies Restgemisch gebildet wird, dass zumindest das Restgemisch oder ein Teil hiervon bei der Bildung des Reaktionseinsatzes verwendet wird, und dass der Reaktionseinsatz derart gebildet wird, dass ein molares Verhältnis von Wasserstoff zu Kohlenmonoxid in dem Reaktionseinsatz weniger als 2 beträgt und der Reaktionseinsatz 2 bis 10 Molprozent Kohlendioxid aufweist.

2. Verfahren nach Anspruch 1, bei dem das Zwischengemisch derart gebildet wird, dass es einen Gehalt an Kohlendioxid von mehr als 95 Molprozent, einen Gehalt an Dimethylether von weniger als 1 Molprozent und einen Gehalt von Wasser und Methanol von weniger als 0,1 Molprozent aufweist.

3. Verfahren nach einem der vorstehenden Ansprüche, bei dem bei der Bildung des Reaktionseinsatzes ferner ein Wasserstoff und Kohlenmonoxid enthaltender und an Kohlendioxid armer oder freier Frischeinsatz verwendet wird, wobei ein molares Verhältnis von Wasserstoff zu Kohlenmonoxid in dem Frischeinsatz 1,0 bis 1,8 beträgt.

4. Verfahren nach Anspruch 3, bei dem der Frischeinsatz mittels eines oder mehrerer Hüttenwerksprozesse (10) bereitgestellt wird.

5. Verfahren nach einem der vorstehenden Ansprüche, bei dem in der Trennung eine überwiegend oder ausschließlich Methanol enthaltende Methanolfraktion gebildet und bei der Bildung des Reaktionseinsatzes verwendet wird.

6. Verfahren nach einem der vorstehenden Ansprüche, bei dem die Kohlendioxidfraktion derart gebildet wird, dass sie wenigstens 99 Molprozent Kohlendioxid aufweist.

7. Verfahren nach einem der vorstehenden Ansprüche, bei dem die Abtrennung zumindest des überwiegenden Anteils des Kohlendioxids aus dem Zwischengemisch oder dessen Anteil unter Verwendung einer Kohlendioxidabtrennkolonne (C5) oder durch Entspannung des Zwischengemischs oder dessen Anteils durchgeführt wird.

8. Verfahren nach Anspruch 7, bei dem die Kohlendioxidabtrennkolonne (C5) mit einem Sumpfverdampfer und ohne einen Kopfkondensator betrieben wird und/oder bei dem als die Kohlendioxidabtrennkolonne (C5) eine Trennkolonne verwendet wird, die 5 bis 20 theoretische Trennböden aufweist.

9. Verfahren nach einem der vorstehenden Ansprüche, bei dem der Trenneinsatz in der Trennung in einer ersten Trennkolonne (C1) bearbeitet wird, in der eine an, Kohlendioxid, Kohlenmonoxid und Wasserstoff abgereicherte und Wasser und Methanol enthaltende erste Sumpfflüssigkeit und ein an, Kohlendioxid, Kohlenmonoxid und Wasserstoff angereichertes und an Wasser und Methanol armes oder freies erstes Kopfgas gebildet werden.

10. Verfahren nach Anspruch 8, bei dem die erste Sumpfflüssigkeit oder ein Teil hiervon in der Trennung in einer zweiten Trennkolonne (C2) bearbeitet wird, in der eine Wasser und Methanol enthaltende und an Dimethylether, Kohlendioxid, Kohlenmonoxid und Wasserstoff arme oder freie zweite Sumpfflüssigkeit und ein Dimethylether, Kohlendioxid, Kohlenmonoxid und Wasserstoff enthaltendes und an Wasser und Methanol armes oder freies zweites Kopfgas gebildet werden.

11. Verfahren nach Anspruch 9, bei dem die zweite Sumpfflüssigkeit oder ein Teil hiervon in der Trennung in einer dritten Trennkolonne (C3) bearbeitet wird, in der eine überwiegend oder ausschließlich Wasser enthaltende dritte Sumpfflüssigkeit und ein überwiegend oder ausschließlich Methanol enthaltendes drittes Kopfgas gebildet werden.

12. Verfahren nach Anspruch 10, bei dem das erste Kopfgas oder ein Teil hiervon unter Bildung von Kondensaten einer stufenweisen Abkühlung unterworfen wird, wobei ein bei der stufenweisen Abkühlung des Kopfgases oder seines Anteils gasförmig verbleibender Rest oder ein Teil hiervon bei der Bildung des Reaktionseinsatzes verwendet wird.

13. Verfahren nach Anspruch 10 oder 11, bei dem die Kondensate oder ein Anteil hiervon und das zweite Kopfgas oder ein Anteil hiervon in einer vierten Trennkolonne (C4) bearbeitet werden, in der ein Kohlendioxid, Kohlenmonoxid und Wasserstoff enthaltendes und an Dimethylether armes oder freies viertes Kopfgas und eine überwiegend oder ausschließlich Dimethylether enthaltende vierte Sumpfflüssigkeit gebildet werden.

14. Verfahren nach Anspruch 12, bei dem ein Teil des vierten Kopfgases kondensiert und zu einem Teil auf die vierte Trennkolonne zurückgeführt und zu einem Teil als das Zwischengemisch (aus dem das CO2 Produkt gewonnen wird) verwendet wird, wobei ein nicht kondensierter Teil des vierten Kopfgases oder ein Teil hiervon bei der Bildung des Reaktionseinsatzes verwendet wird.

## Claims

1. A method (100) for the production of dimethyl ether, wherein a reaction feed containing hydrogen and carbon monoxide is formed, and the reaction feed is subjected to a dimethyl ether direct synthesis to give a raw product containing dimethyl ether, water, methanol, carbon dioxide, carbon monoxide and hydrogen, wherein the raw product or a portion thereof as a separation feed is subjected to a separation in which, during removal of at least the predominant portion of dimethyl ether, the water and the methanol and a lesser portion of the carbon monoxide and hydrogen from the separation feed, an intermediate mixture, which contains carbon dioxide, carbon monoxide and hydrogen and which is low in or free of dimethyl ether, water and methanol, is formed, wherein the separation in which the intermediate mixture is formed is performed purely by condensation and/or distillation, **characterized in that** the intermediate mixture in the separation is provided in liquid form, that during removal of at least the predominant portion of the carbon dioxide from the intermediate mixture or a portion thereof, and while obtaining a carbon dioxide fraction that predominantly or exclusively contains carbon dioxide, a residual mixture is formed that contains carbon monoxide and hydrogen and which is low in or free of carbon dioxide, that at least the residual mixture or a portion thereof is used in the formation of the reaction feed, and that the reaction feed is formed such that a molar ratio of hydrogen to carbon monoxide in the reaction feed is less than 2 and the reaction feed has 2 to 10 mole percent carbon dioxide.

2. The method according to Claim 1, wherein the intermediate mixture is formed to have a carbon dioxide content greater than 95 mole percent, a dimethyl ether content less than 1 mole percent, and a water and methanol content less than 0.1 mole percent.

3. The method according to any one of the preceding claims, wherein a fresh feed, which contains hydrogen and carbon monoxide and which is low in or free of carbon dioxide, is used in the formation of the reaction feed, wherein a molar ratio of hydrogen to carbon monoxide in the fresh feed is 1.0 to 1.8.

4. The method according to Claim 3, wherein the fresh feed is provided by means of one or more smelting processes (10).

5. The method according to any one of the preceding claims, wherein in the separation a methanol fraction containing predominantly or solely methanol is formed and used in the formation of the reaction feed.

6. The method according to any one of the preceding claims, wherein the carbon dioxide fraction is formed to have at least 99 mole percent carbon dioxide.

7. The method according to any one of the preceding claims, wherein the removal of at least the predominant fraction of the carbon dioxide from the intermediate mixture or the proportion thereof is performed using a carbon dioxide removal column (C5) or by release of the intermediate mixture or portion thereof.

8. The method according to Claim 7, wherein the carbon dioxide separation column (C5) is operated with a sump evaporator and without a top condenser and/or in which a separation column having 5 to 20 theoretical separation trays is used as the carbon dioxide separation column (C5).

9. The method according to any one of the preceding claims, wherein the separation feed is processed in the separation in a first separation column (C1) in which a first sump liquid depleted of carbon dioxide, carbon monoxide and hydrogen and containing water and methanol, and a first top gas, which is enriched in carbon dioxide, carbon monoxide and hydrogen and which is low in or free of water and methanol, are formed.

10. The method according to Claim 8, wherein the first sump liquid or a portion thereof is processed in the separation in a second separation column (C2) in which a second sump liquid, which contains water and methanol and which is low in or free of dimethyl ether, carbon dioxide, carbon monoxide and hydrogen, and a second top gas, which contains water and methanol and which is low in or free of water and methanol, are formed.

11. The method according to Claim 9, wherein the second sump liquid or a portion thereof is processed in the separation in a third separating column (C3) in which a third sump liquid containing predominantly or exclusively water and a third top gas containing predominantly or exclusively methanol are formed.

12. The method according to Claim 10, wherein the first top gas or a portion thereof is subjected to step-wise cooling to form condensates, wherein a residue, or a portion thereof, which remains gaseous during the step-wise cooling of the top gas or its portion, is used in the formation of the reaction feed.

13. The method according to Claim 10 or 11, wherein the condensates or a portion thereof and the second top gas or a portion thereof are processed in a fourth separation column (C4) in which a fourth top gas, which contains carbon dioxide, carbon monoxide and hydrogen and which is low in or free of dimethyl ether, and a fourth sump liquid containing predominantly or exclusively dimethyl ether, are formed.

14. The method according to Claim 12, wherein a portion of the fourth top gas condenses and is partially recycled to the fourth separation column and partially used as the intermediate mixture (from which the CO2 product is recovered), wherein a non-condensed portion of the fourth top gas or a portion thereof is used in the formation of the reaction feed.

## Revendications

1. Procédé (100) d'obtention de diméthyléther, dans lequel un insert de réaction contenant de l'hydrogène et du monoxyde de carbone est formé et l'insert de réaction est soumis à une synthèse directe de diméthyléther pour obtenir un produit brut contenant du diméthyléther, de l'eau, du méthanol, du dioxyde de carbone, du monoxyde de carbone et de l'hydrogène, le produit brut ou une partie de celui-ci étant soumis comme insert de séparation à une séparation, dans laquelle, par la séparation d'au moins une partie majeure du diméthyléther, de l'eau et du méthanol, ainsi que d'une partie mineure du dioxyde de carbone et de l'hydrogène, un mélange intermédiaire contenant du dioxyde de carbone, du monoxyde de carbone et de l'hydrogène et pauvre en diméthyléther, eau et hydrogène est formé à partir de l'insert de séparation, la séparation, dans laquelle le mélange intermédiaire est formé, étant réalisée purement par condensation et/ou par distillation, **caractérisé en ce que** le mélange intermédiaire est fourni sous forme liquide dans la séparation, que, par la séparation d'au moins la partie majeure du dioxyde de carbone du mélange intermédiaire ou d'une partie de celui-ci et par l'obtention d'une fraction de dioxyde de carbone contenant principalement ou exclusivement du dioxyde de carbone, un mélange résiduel contenant du monoxyde de carbone et de l'hydrogène et pauvre en ou exempt de dioxyde de carbone est formé, qu'au moins le mélange résiduel ou une partie de celui-ci est utilisé dans la formation de l'insert de réaction et que l'insert de réaction est formé de telle façon qu'un rapport molaire de l'hydrogène au monoxyde de carbone dans l'insert de réaction soit inférieur à 2 et que l'insert de réaction présente de 2 à 10 % en moles de dioxyde de carbone.

2. Procédé selon la revendication 1, dans lequel le mélange intermédiaire est formé de telle façon qu'il présente une teneur en dioxyde de carbone supérieure à 95 % en moles, une teneur en diméthyléther inférieure à 1 % en moles et une teneur en eau et méthanol inférieure à 0,1 % en moles.

3. Procédé selon l'une quelconque des revendications précédentes, dans lequel, lors de la formation de l'insert de réaction, un insert frais contenant de l'hydrogène et du monoxyde de carbone et pauvre en ou exempt de dioxyde de carbone est en outre utilisé, un rapport molaire de l'hydrogène au monoxyde de carbone dans l'insert frais étant de 1,0 à 1,8.

4. Procédé selon la revendication 3, dans lequel l'insert frais est fourni au moyen d'un ou plusieurs processus sidérurgiques (10).

5. Procédé selon l'une quelconque des revendications précédentes, dans lequel une fraction de méthanol contenant principalement ou exclusivement du méthanol est formée dans la séparation et est utilisée dans la formation de l'insert de réaction.

6. Procédé selon l'une quelconque des revendications précédentes, dans lequel la fraction de dioxyde de carbone est formée de telle façon qu'elle présente au moins 99 % en moles de dioxyde de carbone.

7. Procédé selon l'une quelconque des revendications précédentes, dans lequel la séparation d'au moins la partie majeure du dioxyde de carbone du mélange intermédiaire ou d'une partie de celui-ci est effectuée en utilisant une colonne de séparation de dioxyde de carbone (C5) ou par la détente du mélange intermédiaire ou de la partie de celui-ci.

8. Procédé selon la revendication 7, dans lequel la colonne de séparation de dioxyde de carbone (C5) fonctionne avec un évaporateur de puits ou sans un condensateur de tête et/ou dans lequel une colonne de séparation présentant 5 à 20 fonds de séparation théoriques est utilisée comme la colonne de séparation de dioxyde de carbone (C5).

9. Procédé selon l'une quelconque des revendications précédentes, dans lequel l'insert de séparation est traité, lors de la séparation, dans une première colonne de séparation (C1), dans laquelle un premier liquide de fond enrichi en dioxyde de carbone, monoxyde de carbone et hydrogène et contenant de l'eau et du méthanol et un premier gaz de tête enrichi en dioxyde de carbone, monoxyde de carbone et hydrogène et pauvre en ou exempt d'eau et de méthanol sont formés.

10. Procédé selon la revendication 8, dans lequel le premier liquide de fond ou une partie de celui-ci est traité dans une deuxième colonne de séparation (C2), dans laquelle un deuxième liquide de fond contenant de l'eau et du méthanol et pauvre en ou exempt de diméthyléther, dioxyde de carbone, monoxyde de carbone et hydrogène et un deuxième gaz de tête contenant du diméthyléther, du dioxyde de carbone, du monoxyde de carbone et de l'hydrogène et pauvre en ou exempt d'eau et de méthanol sont formés.

11. Procédé selon la revendication 9, dans lequel le deuxième liquide de fond ou une partie de celui-ci est traité dans une troisième colonne de séparation (C3), dans laquelle un troisième liquide de fond contenant principalement ou exclusivement de l'eau et un troisième gaz de tête contenant principalement ou exclusivement du méthanol sont formés.

12. Procédé selon la revendication 10, dans lequel le premier gaz de tête ou une partie de celui-ci est soumis à un refroidissement graduel avec formation de condensats, un résidu gazeux restant lors du refroidissement graduel du gaz de tête ou de la partie de celui-ci, ou une partie de celui-ci étant utilisé lors de la formation de l'insert de réaction.

13. Procédé selon la revendication 10 ou 11, dans lequel les condensats ou une partie de ceux-ci et le deuxième gaz de tête ou une partie de celui-ci sont traités dans une quatrième colonne de séparation (C4), dans laquelle un quatrième gaz de tête contenant du dioxyde de carbone, du monoxyde de carbone et de l'hydrogène et pauvre en ou exempt de diméthyléther et un quatrième liquide de fond contenant principalement ou exclusivement du diméthyléther sont formés.

14. Procédé selon la revendication 12, dans lequel une partie du quatrième gaz de tête est condensée et en partie retournée à la quatrième colonne de séparation et en partie utilisée comme le mélange intermédiaire (à partir duquel le produit de CO2 est obtenu), une partie non condensée du quatrième gaz de tête ou une partie de celle-ci étant utilisée lors de la formation de l'insert de réaction.
